# EUROPEAN PATENT APPLICATION

(11) **EP 4 559 465 A1**
(43) Date of publication of application: **28.05.2025**
(21) Application number: 23842372.7
(22) Date of filing: 19.07.2023
(51) Int. Cl.: A61K 31/496, A61P 13/12

(54) **USE OF OVATODIOLIDE DERIVATIVE AND PHARMACEUTICAL COMPOSITION THEREOF IN PREVENTION AND TREATMENT OF RENAL FIBROSIS**

(30) Priority: 20.07.2022 CN 202210861718
(71) Applicant: Zhujiang Hospital of Southern Medical University, Guangzhou, Guangdong 510280 (CN); Accendatech Hefei Biotech Co., Ltd., Hefei, Anhui 230088 (CN)
(72) Inventor: LONG, Haibo, Hefei, Anhui 230088 (CN); BAO, Shiqi, Hefei, Anhui 230088 (CN)
(74) Representative: Gille Hrabal Partnerschaftsgesellschaft mbB Patentanwälte
(86) International application number: PCT/CN2023/108239
(87) International publication number: WO 2024/017310

(57) **Abstract**

The present invention relates to use of a compound represented by formula (I) or a pharmaceutically acceptable salt thereof in the preparation of a medicament for treating and/or preventing renal fibrosis and/or renal diseases related to renal fibrosis. The compound represented by formula (I) has a structure as follows:

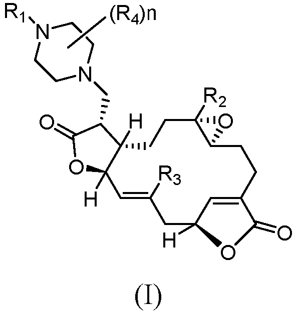

## Description

The present application claims priority to the prior application with the patent application No. 202210861718.4 and entitled "USE OF OVATODIOLIDE DERIVATIVE AND PHARMACEUTICAL COMPOSITION THEREOF IN PREVENTION AND TREATMENT OF RENAL FIBROSIS" filed with the China National Intellectual Property Administration on July 20, 2022, which is incorporated herein by reference in its entirety.

### TECHNICAL FIELD

The present disclosure relates to the field of medicinal chemistry, and in particular, relates to use of an ovatodiolide derivative and a pharmaceutical composition thereof in the prevention and treatment of renal fibrosis (RF) and/or kidney diseases related thereto.

### BACKGROUND

Chronic kidney disease (CKD) is a global major public health problem that seriously harms human health, affecting up to 10%-15% of the population. At present, in China, there are about 130 million adult patients with CKD. Moreover, with the increase in diabetes, hypertension, obesity, and aging population, the morbidity of CKD is increasing year by year around the world. The prevalence of CKD in all age groups worldwide increased by 29.3% from 1990 to 2017, and CKD has become a growing health and social burden.

Regardless of the primary disease of the kidney, once CKD develops, it will continue to progress, ultimately leading to end-stage renal disease (ESRD). ESRD pathologically manifests as RF. RF is the common pathological final result of all kinds of CKD (including primary and secondary glomerular diseases, renal tubular, interstitial and vascular diseases, chronic rejection lesions of transplanted kidneys, etc.) progressing to end-stage renal disease (ESRD). It is mainly manifested by massive deposition of extracellular matrix (ECM), glomerulosclerosis and renal tubular atrophy, suggesting that the renal injury response has entered the final pathway marked by tissue remodeling and loss of organ function.

To date, there is almost no approved treatment specific to RF, other than etiology and diet therapy.
(1) Clinically, the existing anti-RF treatment strategies are mainly based on hemodynamic mechanisms and the reduction of glomerular filtration pressure and include renin-angiotensin-aldosterone system (RAAS) blockers and sodium-glucose co-transporter (SGLT)-2 inhibitors. However, although RAAS blockers can delay the progression of CKD, they cannot completely block the progression and are easy to induce hyperkalemia, acute kidney injury, and the like, thus being unable to achieve satisfactory outcomes; although SGLT-2 inhibitors have certain renal protective effects, their application is limited in the middle and later stages (stages 3b-5) of CKD.
(2) Over the past decade, many anti-RF treatments based on molecular mechanisms have been developed, including treatments targeting proliferation and dysfunction of vascular endothelial cells (endothelin receptor antagonists, etc.), infiltration of inflammatory cells and inflammatory microenvironment (CCL2 selective inhibitors, etc.), epithelial-mesenchymal transdifferentiation (EMT) and related secretory phenotypes of renal tubular epithelial cells (RTECs) (NF-κB inhibitors, etc.), and fibroblast activation (transforming growth factor-β1 antagonists, etc.), but the clinical trials of these treatments have essentially ended with failure.

As such, once CKD patients develop RF, they will inevitably progress toward ESRD and eventually need to rely on dialysis treatment or kidney transplantation for survival, which imposes a great economic burden on their families and society. Therefore, how to effectively prevent and treat RF has become a common concern of the global medical community, and it is of great significance to actively research and develop new drugs for RF treatment that have significant curative effects, minimal toxic and side effects, controllable quality, and convenient use.

At present, no report is found concerning the use of the ovatodiolide derivative of formula (I) or the pharmaceutical composition thereof provided by the present disclosure for the prevention and treatment of RF and/or kidney diseases related thereto.

### SUMMARY

In order to improve the technical solutions existing in the prior art, the present disclosure provides use of a compound of formula (I) or a pharmaceutically acceptable salt thereof for the manufacturing of a medicament for the treatment and/or prevention of renal fibrosis and/or a kidney disease related thereto, wherein the compound of formula (I) is of the structure shown below:

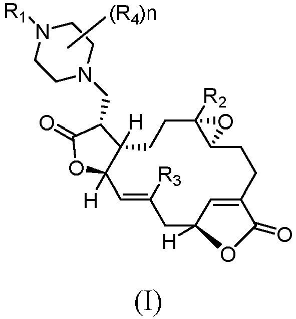

In the formula (I):
the R₁, R₂, and R₃ are each independently selected from H, halogen, C₁₋₆ alkyl, and C₁₋₆ alkoxy.

Each R₄ is independently selected from halogen, C₁₋₆ alkyl, and C₁₋₆ alkoxy; n is selected from 0, 1, 2, 3, and 4.

According to an embodiment of the present disclosure, preferably, the R₁, R₂, and R₃ are selected from methyl, ethyl, and propyl.

According to an embodiment of the present disclosure, the pharmaceutically acceptable salt includes an acid addition salt formed from the compound of formula (I) with an inorganic acid such as hydrochloric acid, hydrofluoric acid, hydrobromic acid, hydroiodic acid, sulfuric acid, pyrosulfuric acid, phosphoric acid or nitric acid; a bisulfate; or an acid addition salt formed from the compound of formula (I) with an organic acid such as formic acid, acetic acid, acetoacetic acid, pyruvic acid, trifluoroacetic acid, propionic acid, butyric acid, caproic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid, p-toluenesulfonic acid, methanesulfonic acid, 2-naphthalenesulfonic acid, naphthalene disulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, hemisulfuric acid or thiocyanic acid.

Preferably, the compound of formula (I) is of the structure shown below:

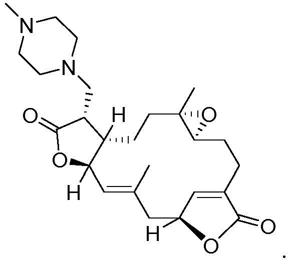

More preferably, the pharmaceutically acceptable salt of the compound of formula (I) is of the structure shown below:

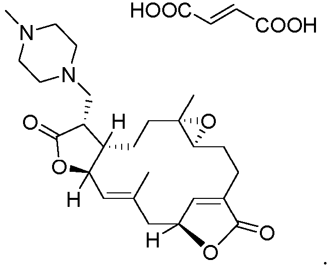

According to an embodiment of the present disclosure, the kidney disease is selected from diabetic nephropathy, primary and secondary glomerulonephritis, hereditary kidney diseases, acute kidney injury, chronic renal failure, lupus nephritis, renal vasculitis, glomerulosclerosis, hypertensive nephropathy (nephrosclerosis), interstitial nephritis, autosomal dominant polycystic kidney disease, Alport syndrome, analgesic nephropathy, and renal allograft injury associated with ischemia-reperfusion or rejection reactions.

In some embodiments, the kidney disease is selected from a variety of primary and secondary glomerular diseases, tubulointerstitial diseases, hereditary kidney diseases, renal vascular diseases, acute kidney injury, chronic renal failure, and renal allograft injury associated with ischemia-reperfusion or rejection reactions, etc.

In some embodiments, the medicament comprises 0.1 wt%-99 wt%, preferably 0.5 wt%-90 wt%, of the compound of formula (I) or the pharmaceutically acceptable salt thereof.

In some embodiments, the medicament further comprises a pharmaceutically acceptable carrier and/or excipient.

In some embodiments, the pharmaceutical composition further comprises one or more additional prophylactic/therapeutic drugs.

In some embodiments, the medicament may be administered in both injectable and oral forms, the injectable forms including intravenous injection and intramuscular injection; the medicament may be manufactured into an oral formulation, an injectable formulation, a topical formulation, etc., such as an injection, a tablet, a pill, a capsule, and the like.

According to an embodiment of the present disclosure, the medicament may be manufactured in a single-dose dosage form or a multiple-dose dosage form.

The term "renal fibrosis" in the present disclosure is equivalent to "kidney fibrosis".

### Beneficial Effects of Present Disclosure

The present disclosure provides a compound of formula (I) or a pharmaceutically acceptable salt thereof for manufacturing a medicament for the prevention/treatment of renal fibrosis and/or kidney diseases related thereto. It is proved by assays that the compound ACT004 of the present disclosure can, by regulating the expression of RALB, inhibit the activation of the transforming growth factor (TGF)-β1/Smad, STAT3, and NF-κB signaling pathways and suppress the EMT process in renal tubular epithelial cells, thus achieving the effects of delaying renal fibrosis and further preventing and treating kidney diseases related thereto.

### BRIEF DESCRIPTION OF THE DRAWINGS

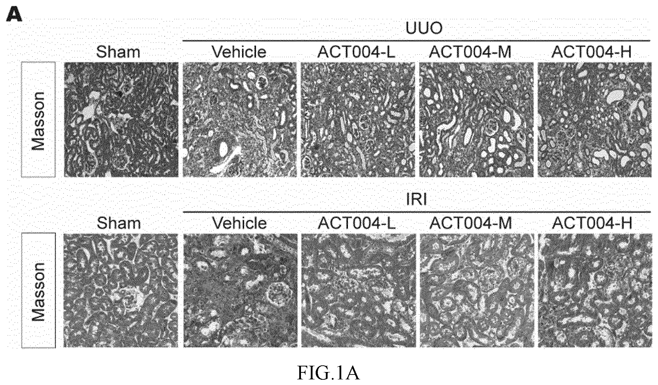

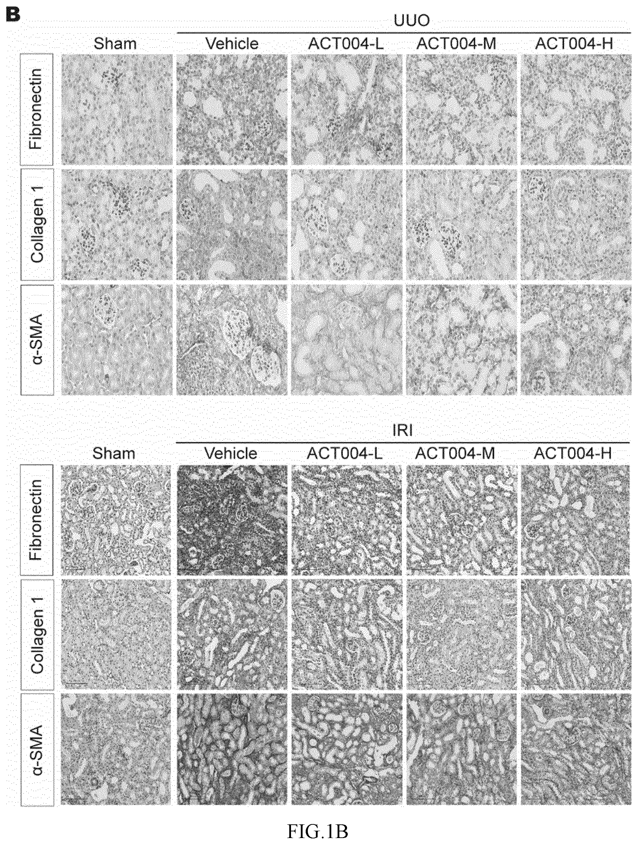

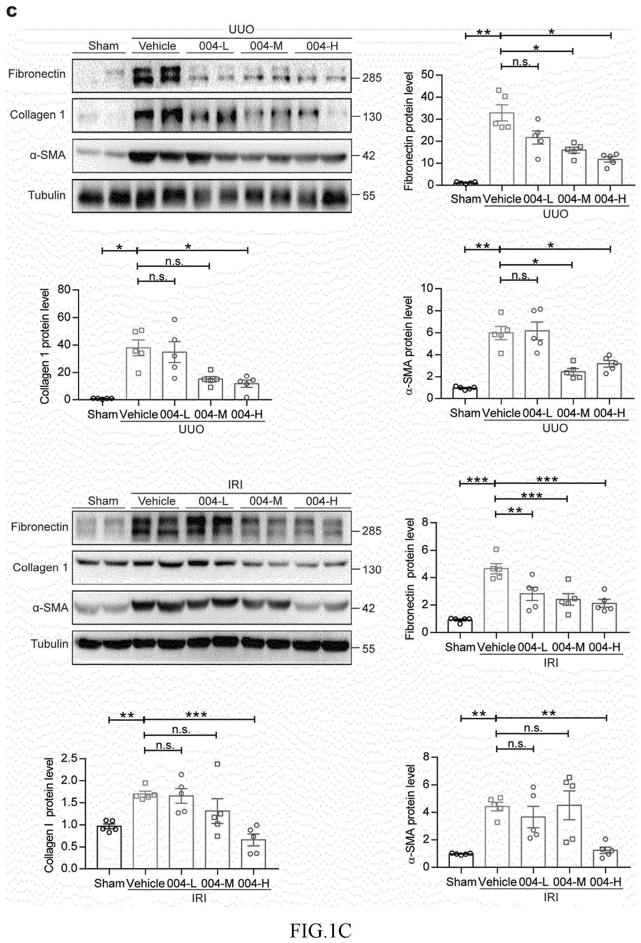

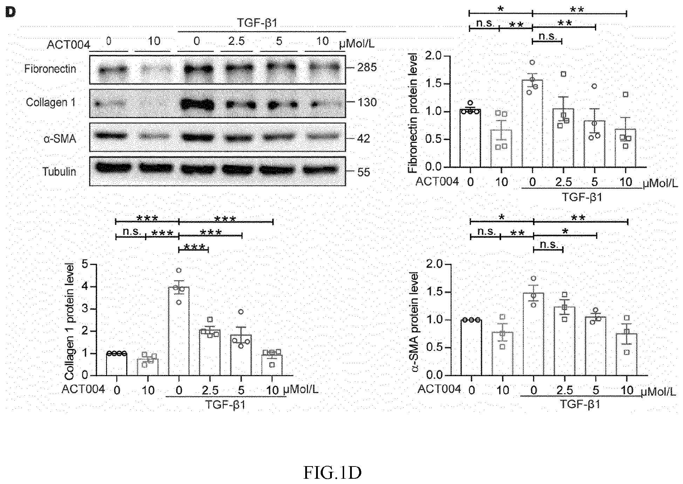

FIG. 1 illustrates the alleviation of renal fibrosis in UUO and IRI models by ACT004, where A shows the Masson staining of kidney tissues from UUO and IRI models; B shows the immunohistochemical staining of kidney tissues from UUO and IRI models treated with ACT004 for detection of the expression of Fibronectin, Collagen I, and α-SMA; C shows the protein expression levels of Fibronectin, Collagen I, and α-SMA in kidney tissues from UUO and IRI models treated with ACT004, as detected by the Western blot method, along with the corresponding statistical diagrams; and D shows the protein expression levels of Fibronectin, Collagen I, and α-SMA in TGFβ1-induced renal tubular epithelial cells treated with different doses of ACT004, as detected by the Western blot method, along with the corresponding statistical diagrams (*, p < 0.05).

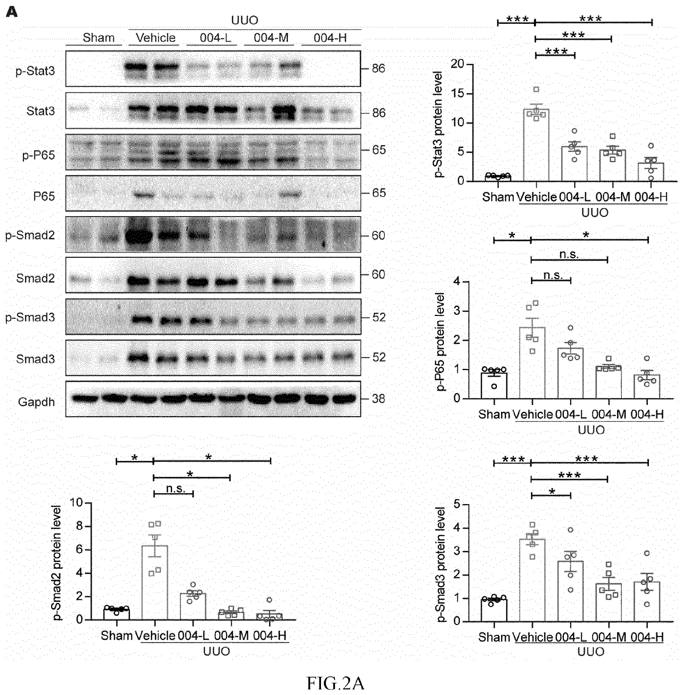

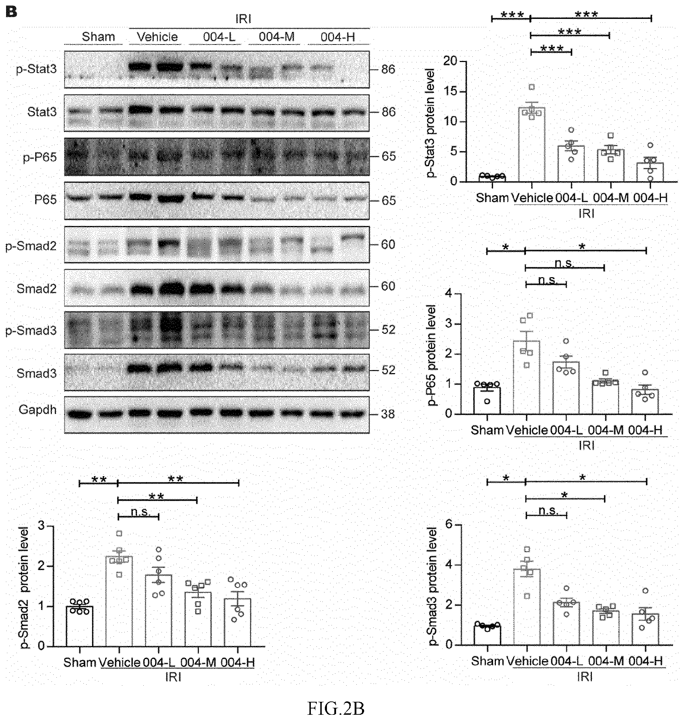

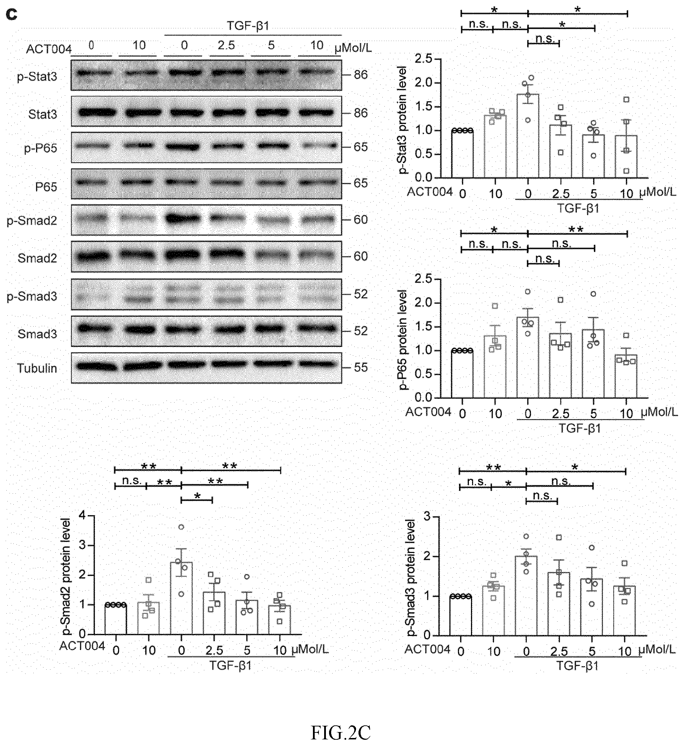

FIG. 2 illustrates the alleviation of renal fibrosis by ACT004 through inhibition of the TGF-β1/Smad, STAT3, and NF-κB signaling pathways, where A shows the protein expression levels of p-Smad2, p-Smad3, p-Stat3, and p-NF-κB in kidney tissues from a UUO model treated with ACT004, as detected by the Western blot method, along with the corresponding statistical diagrams; B shows the protein expression levels of p-Smad2, p-Smad3, p-Stat3, and p-NF-κB in kidney tissues from an IRI model treated with ACT004, as detected by the Western blot method, along with the corresponding statistical diagrams; and C shows the protein expression levels of p-Smad2, p-Smad3, p-Stat3, and p-NF-κB in renal tubular epithelial cells treated with different concentrations of ACT004, with or without TGF-β1 induction for 1 h, as detected by the Western blot method, along with the corresponding statistical diagrams (*, p < 0.05; **, p < 0.01; ***, p < 0.001).

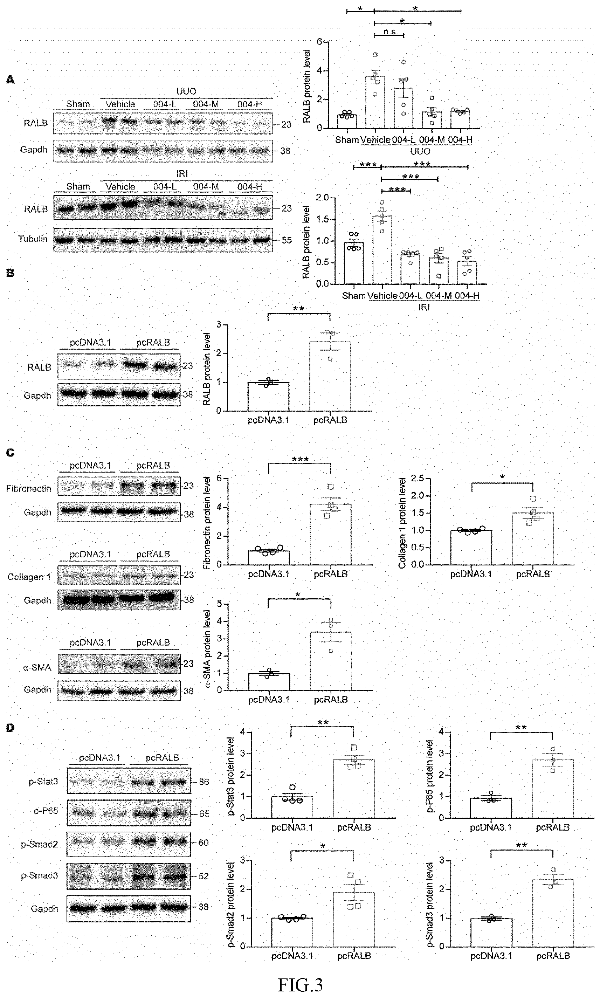

FIG. 3 illustrates the process of RALB promoting renal fibrosis and EMT of renal tubular epithelial cells, where A shows the protein expression levels of RALB in kidney tissues from UUO and IRI models treated with ACT004, as detected by the Western blot method, along with the corresponding statistical diagrams; B shows the efficiency in a cell strain stably over-expressing RALB; C shows the protein expression levels of Fibronectin, Collagen I, and α-SMA in renal tubular epithelial cells stably over-expressing RALB, as detected by the Western blot method, along with the corresponding statistical diagrams; and D shows the protein expression levels of p-Smad2, p-Smad3, p-Stat3, and p-NFKB in renal tubular epithelial cells stably over-expressing RALB, as detected by the Western blot method, along with the corresponding statistical diagrams (*, p < 0.05; **, p < 0.01; ***, p < 0.001).

### DETAILED DESCRIPTION

The technical solutions of the present disclosure will be further described in detail with reference to the following specific examples. It will be appreciated that the following examples are merely exemplary illustrations and explanations of the present disclosure and should not be construed as limiting the claimed scope of the present disclosure. All techniques implemented on the basis of the content described above of the present disclosure are encompassed within the claimed scope of the present disclosure. Unless otherwise stated, the starting materials and reagents used in the following examples are all commercially available products or can be prepared by using known methods.

### Example 1: Compound Preparation

The compound of formula (I) or the pharmaceutically acceptable salt thereof of the present disclosure could be prepared with reference to CN111303178; a compound with the following structure (designated as ACT004) was obtained by referring to the preparation of compound 10 in that patent: 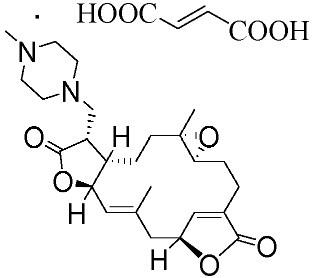 **the chemical name of the compound is** (1a*S*,4*Z*,7*S*,9*E*,10a*S*,13*S*,13a*S*,15a*S*)-9,15a-dimethyl-13-((4-methylpiperazin-1-yl)methyl)-1a,2,3,8,10a,13,13a,14,15,15a-decahydro-5*H*-4,7-(metheno)furo[2,3-*f*]oxireno[2,3-*j*][1]oxacyclopentadecine-5,12(7*H*)-dione fumarate.

### Example 2: Assay for Anti-RF Activity of ACT004

In the initial stage of kidney injury, the production of fibrotic matrix is the mechanism of tissue injury repair. After mild injury, this portion of fibrotic matrix is gradually absorbed without affecting the structure and function of the kidney. However, when CKD occurs, the fibrotic matrix continues to deposit, escaping regulatory suppression, leading to the breakdown of tissue and organ architecture, reduced blood supply, and reduced organ function, which ultimately reduces the kidney tissue repair function and thus results in renal failure. Therefore, the main feature of RF is excessive ECM deposition leading to scar formation, and the pathological manifestation is the proliferation of ECM-producing cells such as fibroblasts and myofibroblasts, so that collagen fibers and fibronectin are excessively produced and accumulated, which progressively develops into glomerulosclerosis and renal interstitial fibrosis, ultimately leading to loss of kidney function.

The complex mechanism of RF generation, regulated by multiple factors, has not been fully elucidated. The chronic inflammatory responses and the renal tubular epithelial-mesenchymal transdifferentiation (TEMT) accompanying the injury repair process are two important mechanisms. Various cells, mediators, growth factors and signal pathways, such as monocyte-macrophages, Notch, Wnt and Hedgehog signaling pathways, TGF-β1, basic fibroblast growth factor, angiotensin II, and the like, directly or indirectly participate in this process. Injury to RTECs is an early event in the initiation of the fibrotic response, and is regulated by numerous factors, including various transcription factors, growth factors, cytokines, hormones, and extracellular signals. Among the numerous profibrotic factors, TGF-β1 is the most prominent growth factor that initiates and drives the development of EMT. It regulates the EMT process through the Smad-dependent classical pathway. RTECs undergoing EMT lose their normal substance transport function, experience G2/M arrest leading to a decrease in proliferation and repair capacity, and exhibit significant secretory phenotype transformation, with significantly up-regulated expression levels of various profibrotic factors including TGF-β1. Through the secreted profibrotic factors, the RTECs undergoing EMT interact with renal interstitial fibroblasts and induce the proliferation and activation of the interstitial fibroblasts, thereby promoting the progression of RF. Selective blocking of TEMT not only can protect the function of RTECs and inhibit the formation of myofibroblasts, but also can significantly reduce the expression levels of various inflammatory factors in the kidney and the infiltration of interstitial inflammatory cells, effectively delaying the RF development in various CKD models.

### 2.1 Experimental materials

### 2.1.1 Test drug

ACT004.

### 2.1.2 Experimental animals

Sixty 8-week-old C57 mice (SPF grade) were supplied by the Laboratory Animal Center of Zhujiang Hospital, Southern Medical University, Guangzhou. Before the experiment, the animals were acclimated to the indoor environment for 7 days at a room temperature of 18-22 °C and a relative humidity of 65%. All mice had free access to water and were fed with regular feed (protein content: 3.14 mg/g).

### 2.1.3 Experimental cell strain

The immortalized mouse-derived renal tubular epithelial cell line was provided by the Professor Nie Xiaoli's research team at the School of Traditional Chinese Medicine, Southern Medical University.

### 2.2 Experimental procedures

### 2.2.1 Therapeutic effects of ACT004 on unilateral ureteral obstruction (UUO) and ischemia-reperfusion injury (IRI) RF animal models

### 2.2.1.1 Animal model establishment and grouping

Method for constructing UUO model: Under anesthesia with intraperitoneal injection of tribromoethanol, the surgical area for abdominal midline incision was shaved and disinfected. After making the abdominal midline incision, the left ureter was located, and the upper segment of the ureter was isolated. A complete ureteral obstruction was induced by double ligation using 4-0 silk sutures (UUO group). For mice in the sham group, the ureter was similarly exposed and isolated, but no ligation was performed. Layered suturing of the abdominal incision was performed, followed by disinfection with iodophor. Mice subjected to UUO surgery were randomly divided into the following 5 groups: a UUO group, a UUO + low-dose ACT004 (12.5 mg/kg.d) group, a UUO + medium-dose ACT004 (25 mg/kg.d) group, a UUO + high-dose ACT004 (50 mg/kg.d) group, and a sham group, with 6 mice in each group. Starting from day 4 after the surgery, intragastric administration was performed. The sham group and the UUO groups were given an equal volume of normal saline as a control treatment. On day 11 after the surgery, the left kidney tissues were collected.

Method for constructing IRI model: Mice were anesthetized intraperitoneally with tribromoethanol. An abdominal midline incision or dorsal incision was made. The left renal vein was isolated, while the renal artery was clamped using an arterial clip. The mice were placed on a constant-temperature pad at 37.5 °C for 30 min, and the incision was covered with PBS-moistened gauze. After 30 min, the arterial clip was released, and the abdomen was closed after the recovery of renal congestion was observed. Mice subjected to IRI surgery were randomly divided into the following 5 groups: an IRI group, an IRI + low-dose ACT004 (12.5 mg/kg.d) group, an IRI + medium-dose ACT004 (25 mg/kg.d) group, an IRI + high-dose ACT004 (50 mg/kg.d) group, and a sham group, with 6 mice in each group. Starting from day 4 after the surgery, intragastric administration was performed. The sham group and the IRI groups were given an equal volume of normal saline as a control treatment. On day 10 after the surgery, the right kidney was removed, and on day 11 day after the surgery, the left kidney tissues were collected.

### 2.2.1.2 Drug dose setting and administration

According to the results of the preliminary experiment, the medium dose of ACT004 for mice was 25 mg/kg.d. Therefore, low-dose (12.5 mg/kg.d), medium-dose (25 mg/kg.d) and high-dose (50 mg/kg.d) groups were set. ACT004 was prepared into a solution using normal saline, and intragastrically administered once daily starting on day 4 after the surgery for a duration of 1 week. Mice in the sham, UUO and IRI groups were subjected to intragastric administration with an equal amount of normal saline for a duration of 1 week.

### 2.2.1.3 Specimen collection and processing

After 1 week of intragastric administration, kidney tissues were collected as follows: Anesthesia was conducted using single intraperitoneal injection of tribromoethanol at a concentration of 200 mg/kg. Blood was collected by enucleation, and serum was separated and stored at -20 °C or -70 °C. An abdominal incision was made, and sterile gauze was used to move aside abdominal organs. Cold PBS was perfused through the heart until the organs turned white. Kidney tissues were then collected. The entire left kidney was weighed, then bisected along the median sagittal plane, and washed thoroughly with PBS, and the capsule was removed. The tissue was then fixed in 10% neutral formaldehyde buffer for subsequent pathological examination.

### 2.2.1.4 Observation indexes and assay method

2.2.1.4.1 Renal pathology: Masson staining was used to observe the renal pathological structures and collagen fiber deposition.

2.2.1.4.2 Protein level detection: Ras GTPase superfamily member RALB, EMT-related index α-SMA, and fibrosis phenotype-related indexes Fibronectin and Collagen I.

### 2.2.2 Effect of ACT004 on TGF-β1-induced EMT in renal tubular epithelial cells

2.2.2.1 Cell model establishment and grouping

Method for constructing RF cell model: Human recombinant TGF-β1 cytokine (10 ng/µL) was used to intervene in renal tubular epithelial cells (NRK) for 48 h, causing NRK to show fibroid changes and increased expression of fibrosis phenotypes (Fibronectin, Collagen I, and α-SMA). The addition of TGF-β1 was accompanied by the administration of different concentrations of ACT004 intervention (2.5 µM, 5 µM, and 10 µM). The cells were divided into the following 6 groups: a blank control group, a blank control + ACT004 (5 µM) group, a TGF-β1 model group, and groups for TGF-B1 + different concentrations of ACT004 intervention (2.5 µM, 5 µM, and 10 µM).

### 2.2.2.2 Observation indexes and assay method

Protein level detection: Ras GTPase superfamily member RALB, EMT-related index α-SMA, and fibrosis phenotype-related indexes Fibronectin and Collagen I.

### 2.2.3 Exploration of relationship between anti-RF effect of ACT004 and RALB protein

### 2.2.3.1 Construction of cell strain stably over-expressing RALB

The Plasmid targeting the RALB sequence, namely pc-RALB, was constructed by Suzhou GenePharma Co., Ltd. (with the empty vector pcDNA3.1 as a negative control) and transfected into NRK.

### 2.2.3.2 Observation indexes and assay method

Protein level detection: RALB, EMT-related index α-SMA, and fibrosis phenotype-related indexes Fibronectin and Collagen I.

### 2.3 Experimental results

### 2.3.1 Pathological changes in mouse kidney tissue

From A of FIG. 1, Masson staining shows that for the UUO and IRI mouse models, the conditions of renal interstitial expansion, extracellular matrix deposition, increased collagen fibers, and inflammatory cell infiltration were significantly reduced in the ACT004 treatment groups; the immunohistochemical staining results for the fibrosis indexes Fibronectin, Collagen I, and α-SMA very objectively demonstrate the inhibitory effect of ACT004 treatment on the expression of Fibronectin, Collagen I, and α-SMA. The Wb bands in panels C and D reflect that ACT004 could function to inhibit the progression of RF at both *in-vivo* and *in-vitro* levels, and the differences were statistically significant.

### 2.3.2 Alleviation of renal fibrosis by ACT004 through effective regulation of TGF-β1/Smad, STAT3, and NF-κB signaling pathways

As can be seen from A and B of FIG. 2, the expression levels of p-Smad2, p-Smad3, p-Stat3, and p-NFκB in kidney tissues were significantly up-regulated in the UUO and IRI models, while these expression levels were significantly reduced in the ACT004 treatment groups. The bands in panel C reflect that the increased expression levels of p-Smad2, p-Smad3, p-Stat3, and p-NFκB in renal tubular epithelial cells induced by TGF-β1 were reduced after ACT004 intervention, and the differences were statistically significant. The above results fully confirm that ACT004 could inhibit the activation of the TGF-β1/Smad, STAT3, and NF-κB signaling pathways in renal fibrosis models both *in vivo* and *in vitro.*

### 2.3.3 Anti-renal fibrosis effect of ACT004 through regulation of RALB expression

As can be seen from A of FIG. 3, the expression level of RALB in kidney tissues was significantly up-regulated in the UUO and IRI models, while this expression level was significantly reduced in the ACT004 treatment groups. The bands in panel B reflect that the level of RALB was significantly up-regulated in the cells of the pcRALB group, indicating the successful construction of this stable over-expression cell strain. Under these conditions, fibrosis indexes (C) and p-Smad2, p-Smad3, p-Stat3 and p-NFκB indexes (D) were detected. The protein levels of the fibrosis indexes Fibronectin, Collagen I and α-SMA, as well as p-Smad2, p-Smad3, p-Stat3 and p-NFκB, were significantly up-regulated, and the differences were statistically significant. The above results positively confirm that *in vitro,* RALB could promote the EMT process in renal tubular epithelial cells and might promote renal fibrosis by activating the TGF-β1/Smad, STAT3, and NF-κB signaling pathways.

### 2.4 Discussion

### 2.4.1 Animal models

At the end of the unilateral ureteral obstruction (UUO) experiment, the animal model showed thin renal cortex and significant hydronephrosis, belonging to a typical obstructive renal fibrosis model. During the ischemia-reperfusion injury (IRI) experiment, after the renal pedicle was clamped with the micro arterial clip, the kidney could be seen to change from bright red to white and then to dark purple, indicating successful clamping; upon removal of the arterial clip, the blood perfusion was restored, and then the kidney could be seen to rapidly change from dark purple to bright red, which is a typical phenomenon of renal ischemia-reperfusion.

### 2.4.2 Therapeutic effects

ACT004 could significantly alleviate the renal fibrosis changes in the UUO and IRI mice, inhibit the synthesis of the renal interstitial matrix proteins Fibronectin, Collagen I and α-SMA, and suppress the EMT in renal tubular epithelial cells. The renal protective function of ACT004 was achieved primarily by inhibiting the activation of the TGF-β1/Smad, STAT3, and NF-κB signaling pathways. ACT004 down-regulated the expression of RALB in kidney tissues from the UUO and IRI models at the *in-vivo* level, and RALB over-expression aggravated renal fibrosis and induced the activation of the TGF-β1/Smad, STAT3, and NF-κB signaling pathways. In summary, ACT004 regulates the expression of RALB to inhibit the activation of the TGF-β1/Smad, STAT3, and NF-κB signaling pathways and suppress the EMT process in renal interstitial tubular epithelial cells, thus achieving the effects of delaying renal fibrosis and further treating kidney diseases related thereto.

The embodiments of the present disclosure have been described above. However, the present disclosure is not limited to the embodiments described above. Any modification, equivalent replacement, improvement, and the like made without departing from the spirit and principle of the present disclosure shall fall within the claimed scope of the present disclosure.

## Claims

1. Use of a compound of formula (I) or a pharmaceutically acceptable salt thereof for the manufacturing of a medicament for the treatment and/or prevention of renal fibrosis and/or a kidney disease related thereto, wherein the compound of formula (I) is of the structure shown below: 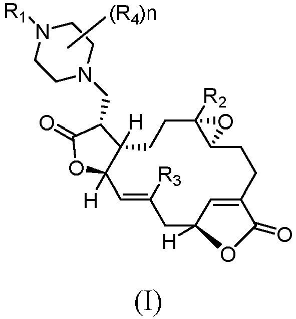 in the formula (I),
the R₁, R₂, and R₃ are each independently selected from H, halogen, C₁₋₆ alkyl, and C₁₋₆ alkoxy;
each R₄ is independently selected from halogen, C₁₋₆ alkyl, and C₁₋₆ alkoxy; n is selected from 0, 1, 2, 3, and 4.

2. The use as claimed in claim 1, wherein the R₁, R₂, and R₃ are selected from methyl, ethyl, and propyl.

3. The use as claimed in claim 1, wherein the pharmaceutically acceptable salt is selected from an acid addition salt formed from the compound of formula I with an inorganic acid such as hydrochloric acid, hydrofluoric acid, hydrobromic acid, hydroiodic acid, sulfuric acid, pyrosulfuric acid, phosphoric acid or nitric acid; a bisulfate; and an acid addition salt formed from the compound of formula I with an organic acid such as formic acid, acetic acid, acetoacetic acid, pyruvic acid, trifluoroacetic acid, propionic acid, butyric acid, caproic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid, p-toluenesulfonic acid, methanesulfonic acid, 2-naphthalenesulfonic acid, naphthalene disulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, hemisulfuric acid or thiocyanic acid.

4. The use as claimed in claim 1, wherein the compound of formula (I) is of the structure shown below:

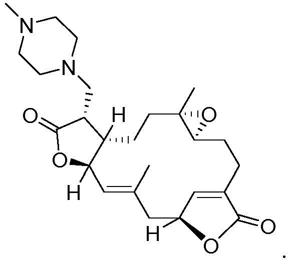

5. The use as claimed in claim 1, wherein the pharmaceutically acceptable salt of the compound of formula (I) is of the structure shown below:

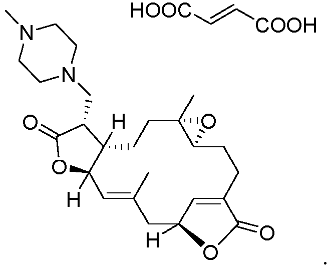

6. The use as claimed in any one of claims 1-5, wherein the kidney disease is selected from a variety of primary and secondary glomerular diseases, tubulointerstitial diseases, hereditary kidney diseases, renal vascular diseases, acute kidney injury, chronic renal failure, and renal allograft injury associated with ischemia-reperfusion or rejection reactions, etc.

7. The use as claimed in any one of claims 1-5, wherein the medicament comprises 0.1 wt%-99 wt%, preferably 0.5 wt%-90 wt%, of the compound of formula I or the pharmaceutically acceptable salt thereof.

8. The use as claimed in any one of claims 1-5, wherein the medicament further comprises a pharmaceutically acceptable carrier and/or excipient.

9. The use as claimed in any one of claims 1-5, wherein the medicament can be administered in both injectable and oral forms, the injectable forms comprising intravenous injection and intramuscular injection.

10. The use as claimed in any one of claims 1-5, wherein the medicament can be manufactured into an oral formulation, an injectable formulation, a topical formulation, etc., such as an injection, a tablet, a pill, or a capsule.
